# EUROPÄISCHE PATENTANMELDUNG

(11) **EP 1 306 053 A2**
(43) Veröffentlichungstag der Anmeldung: **02.05.2003**
(21) Anmeldenummer: 02022892.0
(22) Anmeldetag: 14.10.2002
(51) Int. Cl.: A61B 6/00

(54) **Universal-Radiographie-System**

(30) Priorität: 15.10.2001 DE 10150794
(71) Anmelder: SIEMENS AKTIENGESELLSCHAFT, 80333 München (DE)
(72) Erfinder: Horbaschek, Heinz, 91056 Erlangen (DE); Molz, Claudius, 91054 Buckenhof (DE)

(57) **Zusammenfassung**

Die Erfindung betrifft ein Radiographie-System mit einem ersten deckenaufgehängten System (1) für einen Röntgenstrahler (2), der in den drei Raumkoordinaten (x, y, z) mittels des ersten deckenaufgehängten Systems (1) verfahrbar und um wenigstens zwei Achsen (3, 4) schwenkbar ist, mit einem zweiten, von dem ersten unabhängigen deckenaufgehängten System (5) für einen Strahlungsempfänger (6), der in den drei Raumkoordinaten (x, y, z) mittels des zweiten deckenaufgehängten Systems (5) verfahrbar ist, und mit einem an dem zweiten deckenaufgehängten System (5) angebrachten Tragarm (10), an dem der Strahlungsempfänger (6) um wenigstens zwei Achsen (DHA, DVA, α, β) bewegbar aufgehängt ist, wobei der Röntgenstrahler (2) und der Strahlungsempfänger (6) gegenüber positionierbar sind.

## Beschreibung

Die Erfindung bezieht sich auf ein Radiographie-System mit einem ersten deckenaufgehängten System für einen Röntgenstrahler, der in den drei Raumkoordinaten mittels des ersten deckenaufgehängten Systems verfahrbar und um wenigstens zwei Achsen schwenkbar ist und mit einem zweiten deckenaufgehängten System für einen Strahlungsempfänger, wobei der Röntgenstrahler und der Strahlungsempfänger sind gegenüber positionierbar sind..

Bei Aufnahmen, bei denen der digitale Bildempfänger in einem stationären Patiententisch eingebaut ist, sind Aufnahmen mit lateraler oder beliebiger Strahlungsrichtung nicht möglich. Diese Aufnahmen müssen weiterhin mit Kassetten durchgeführt werden, was unter anderem einen optimierten Workflow behindert.

Beschränkungen hinsichtlich der möglichen Aufnahmen bestehen auch bei den bekannten Radiographie-Systemen, bei denen der Röntgenstrahler und der Bildempfänger gemeinsam an einem C-oder U-förmigen Träger angeordnet sind, das heißt bezüglich ihres Abstandes fest miteinander gekoppelt sind. Diese feste Koppelung begrenzt die mit dem System durchführbaren Untersuchungen auf solche, die mit dem kopplungsbedingt vorgegebenen Abstand der Strahlungsquelle zum Strahlungsempfänger möglich sind. Um mit dem System auch Untersuchungen durchführen zu können, bei denen ein größerer Fokus-Film-Abstand erforderlich ist, beispielsweise bei Thoraxaufnahmen, bei denen der Abstand ca. 1,80 m und mehr betragen muss, wird normalerweise ein zweiter Strahlungsempfänger verwendet, welcher beispielsweise wandseitig angebracht ist, wie dies beispielsweise bei einem Rasterwandgerät der Fall ist. Das heißt es ist sowohl am Träger, als auch am Rasterwandgerät ein Bildempfänger angeordnet, dieser also mithin doppelt vorgesehen. Dies verursacht naturgemäß zusätzliche Kosten, welche sich insbesondere bei Verwendung eines digitalen Strahlungsempfängers in Form eines Bilddetektors bemerkbar machen, welcher erheblich teuerer ist, als eine Kassettenrasterlade. Darüber hinaus sind Anordnungen mit solchen zusätzlichen Rasterbandgeräten aus baulichen Gründen nicht immer einsetzbar.

Der Erfindung liegt daher die Aufgabe zugrunde, ein Universal-Radiographie-System zu schaffen, welches bei gleichzeitiger Senkung der hiermit verbundenen Kosten die Variabilität der mit dem System durchführbaren Untersuchungen vergrößert.

Zur Lösung dieser Aufgabe ist erfindungsgemäß vorgesehen, dass der Strahlungsempfänger, insbesondere ein digitaler Bildempfänger, an einem zweiten, von dem ersten unabhängigen deckenaufgehängten, System jeweils in den drei Raumkoordinaten mittels des zweiten deckenaufgehängten Systems verfahrbar ist und mit einem an dem zweiten deckenaufgehängten System angebrachten Tragarm versehen ist, an dem der Strahlungsempfänger um wenigstens zwei Achsen (DHA, DVA, α, β) bewegbar aufgehängt ist, wobei bevorzugt - universelle Einsatzmöglichkeiten, wenn auch mit gewissen Einstellungsproblemen behaftet, ergeben sich auch bei eine manuellen Bewegung des Röntgenstrahlers und/oder des Bildempfängers - der Röntgenstrahler und der Strahlungsempfänger mit motorischen Antrieben versehen sind und die mit Positionserfassungseinrichtungen versehenen Antriebe des Röntgenstrahlers und des Strahlungsempfängers über eine Einstellungssoftware miteinander gekoppelt sind.

Erfindungsgemäß kann der Strahlungsempfänger um wenigstens zwei horizontale Achsen des Tragarms bewegbar und um die senkrechte Achse des zweiten deckenaufgehängten Systems rotierbar aufgehängt sein.

Durch die laufende Positionserfassung aller Koordinaten und Winkel und eine entsprechende Software kann je nach dem gewünschten Anwendungsfall eine Koppelung oder ein Nachlauf der Systeme unter Einbeziehung der Lagerungs- bzw. Halteeinrichtungen für den Patienten (Patientenlagerungsplatte oder Patientenentenlagerungstisch für Aufnahmen am liegenden oder sitzenden Patienten bzw. Haltegriffe für Aufnahmen am stehenden Patienten, z. B. Thoraxaufnahmen in zwei Ebenen) erfolgen.

Dabei sind Aufnahmen am sitzenden Patienten in beliebigen Einstrahlungswinkeln ebenso möglich, wie "Kontaktaufnahmen" z. B. der Extremitäten, bei denen das zu untersuchende Objekt direkt auf dem Gehäuse des digitalen Strahlungsempfängers positioniert werden kann.

In Weiterbildung der Erfindung kann dabei vorgesehen sein, dass die Deckenaufhängungen des Röntgenstrahlers und des Bildempfängers so ausgebildet sind, dass der Röntgenstrahler und der Bildempfänger - in Gegenüberstellung - um den Patienten rotierbar sind, sodass Bewegungsabläufe möglich sind, wie sie z. B. für die Tomografie notwendig sind. Möglich sind dabei nicht nur, wie es heute aus Kostengründen für die Mechanik üblich ist, lineare Schichtabläufe oder Schichtfiguren, sondern auch beliebige kreisförmige, spiralförmige, zykloidische usw. Schichtabläufe, wobei die erfassten Datensätze zur Rekonstruktion entweder mehrerer planer Schichten (Tomosynthese) dienen können, wobei die Schichtebenen beliebig im Raum liegen können, sondern aber auch für großvolumige 3D Darstellungen herangezogen werden können.

Es versteht sich dabei von selbst, dass zur Durchführung solcher Aufnahmen eine geeignete Patientenlagerungsvorrichtung mit einer einseitig aufgehängten, bzw. abgestützten strahlungstransparenten Lagerungsplatte erforderlich ist.

In Weiterbildung der Erfindung kann vorgesehen sein, dass zur Aufnahme größerer Bereiche - anstelle der bisher hierfür vorgesehenen großen Kassetten - diese Bereiche durch Verschieben des gegenüber dem aufzunehmenden Bereich kleineren Bildempfängers bei gleichzeitiger Verschwenkung des Röntgenstrahlers abgedeckt werden.

Um dabei trapezförmige Verzerrungen zu vermeiden, kann gemäß einem weiteren Merkmal der vorliegenden Erfindung vorgesehen sein, dass die Bildebene des Strahlungsempfängers jeweils entsprechend der Verschwenkung des Zentralstrahls des Röntgenstrahlers mit verkippt wird.

Weitere Vorteile, Merkmale und Einzelheiten der Erfindung ergeben sich aus der nachfolgenden Beschreibung eines Ausführungsbeispiels sowie anhand der Zeichnung. Dabei zeigen:
- Fig. 1: eine schematische Darstellung eines deckenaufgehängten Röntgenstrahlers mit den verschiedenen Bewegungs- und Rotationsachsen,
- Fig. 2: eine schematische Darstellung der erfindungsgemäßen Deckenaufhängung des Strahlungsempfängers,
- Fig. 3: eine schematische Darstellung der Verschwenkung und Verschiebung von Röntgenstrahler und Strahlungsempfänger zur Aufnahme größerer Bereiche,
- Fig. 4: ein abgewandeltes Aufnahmesystem gemäß Fig. 3 mit zusätzlicher Verkippung der Bildebene entsprechend der Verschwenkung des Röntgenstrahlers und
- Fig. 5 bis 8: einige Ausführungsbeispiele typischer Anwendungen des erfindungsgemäßen UniversalRadiographie-Systems für verschiedenartigste Radiografieanwendungen.

Das deckengeführte Aufhängungssystem 1 für einen Röntgenstrahler 2 ermöglicht sowohl eine Verschiebung entlang einer Längsachse (x-Achse) einer Querachse (y-Achse) und einer vertikalen Achse (Höhen- oder z-Richtung) als auch Rotationen oder Drehungen DHA um die horizontale Achse 3 und Rotationen oder Drehungen DVA um die vertikale Achse 4.

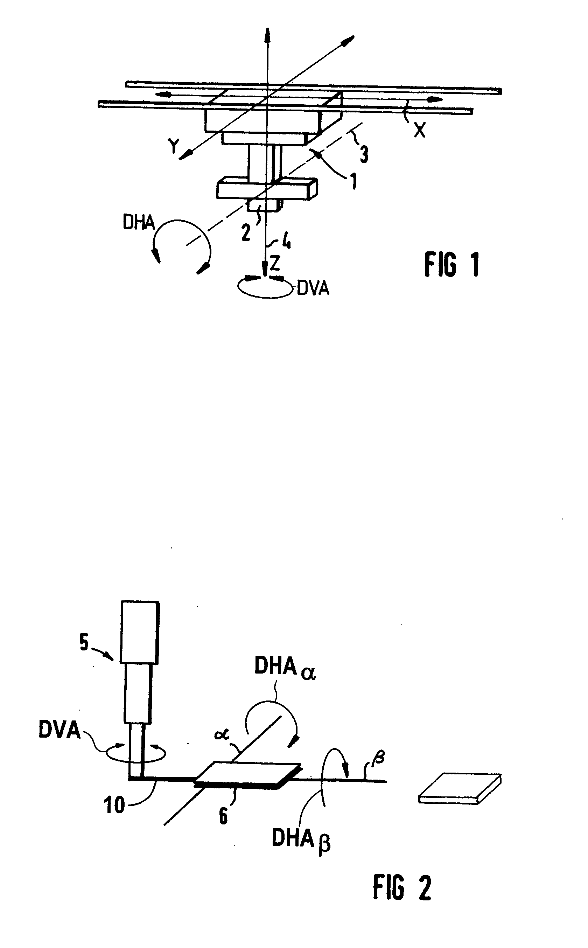

Das in Fig. 2 schematisch dargestellte Deckenaufhängesystem 5 für einen mittels eines Tragarm 10 gehaltenen Strahlungsempfänger 6, insbesondere einen digitalen Bildempfänger, ermöglicht in gleicher Weise sowohl Verschiebungen längs der x-, y- und z-Achse als auch Rotationen oder Drehungen DVA um die vertikale Achse (z-Achse) und darüber hinaus gegenüber dem Tragarm 10 Verschwenkungen um die α-Achse und die β-Achse.

Beide Deckenaufhängesysteme 1 und 5 sollen mit nicht gezeigten motorischen Antrieben versehen sein, die bevorzugt so ausgebildet sind, dass freie Orientierungen des Röntgenstrahlers 1 und des Strahlungsempfängers 6 im Raum möglich sind, wobei die mit Positionserfassungseinrichtungen versehenen Antriebe des Röntgenstrahlers 2 und des Strahlungsempfängers 6 über eine Einstellungssoftware so miteinander gekoppelt sind, dass die für den jeweiligen Anwendungsfall notwendige Zuordnung von Röntgenstrahler und Strahlungsempfänger bei jeder Orientierung automatisch eingestellt wird.

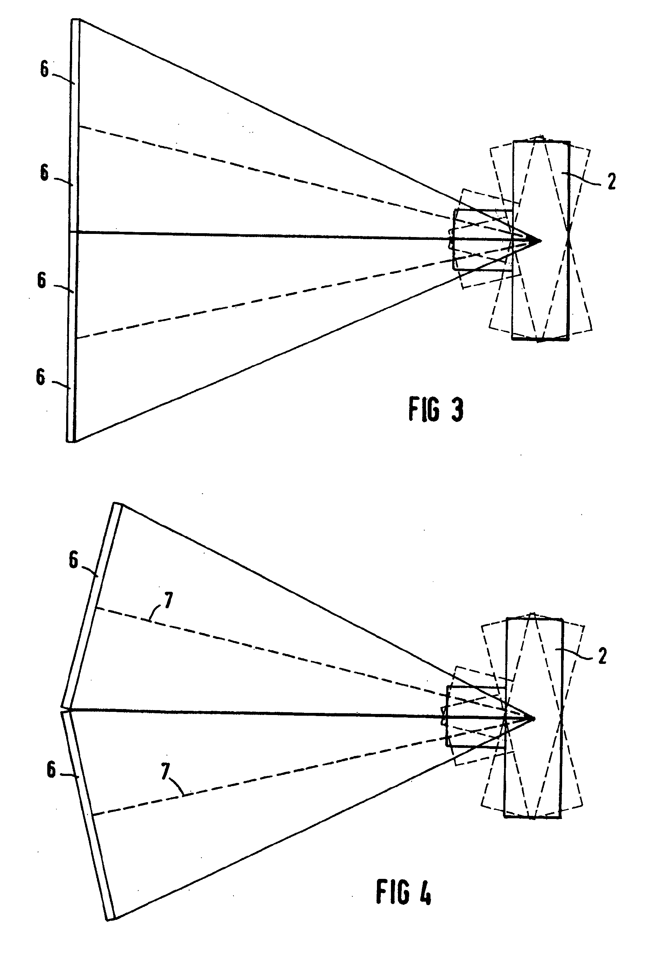

So kann beispielsweise wie in Fig. 3 dargestellt, zum Abdecken größerer Aufnahmebereiche gegenüber der Fläche des Strahlungsempfängers vorgesehen sein, das der Strahlungsempfänger 6 in der Bildebene verschoben wird, während gleichzeitig der Röntgenstrahler 2 verkippt wird. Um dabei trapezförmige Verzerrungen zu vermeiden, kann - wie dies in Fig. 4 schematisch angedeutet ist - mit dem Verkippen des Röntgenstrahlers 2 nicht nur eine Verschiebung des Strahlungsempfängers 6 sondern gleichzeitig auch eine entsprechende Verkippung stattfinden, sodass nach wie vor der Zentralstrahl 7 des Röntgenstrahlers 2 senkrecht auf der Bildebene des Strahlungsempfängers 6 steht.

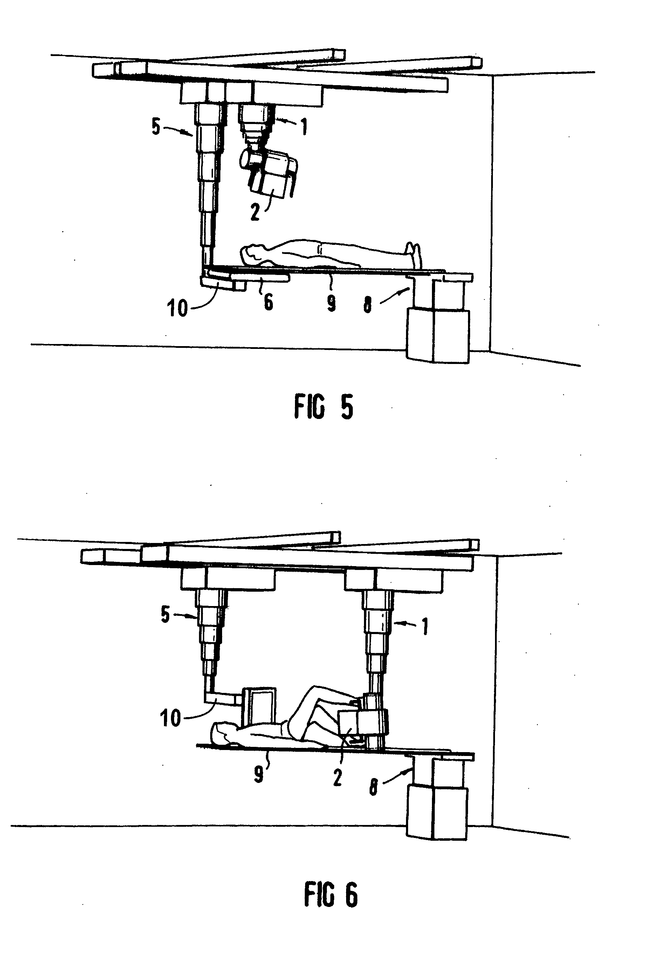

Die Fig. 5 bis 8 zeigen vier unterschiedliche Anwendungsbeispiele für Radiographieaufnahmen mithilfe des erfindungsgemäßen Universal-Systems, unter gleichzeitiger Verwendung einer Patientenlagerungsvorrichtung 8, die eine einseitig abgestützte strahlungstransparente Lagerungsplatte 9 umfasst.

In Fig. 5 ist dabei eine axiale Skull-Aufnahmesituation dargestellt, während Fig. 6 die durch entsprechende Verschiebung der Deckenaufhängesysteme 1 und 5 einstellbare sogenannte Femoral neck axial zeigt.

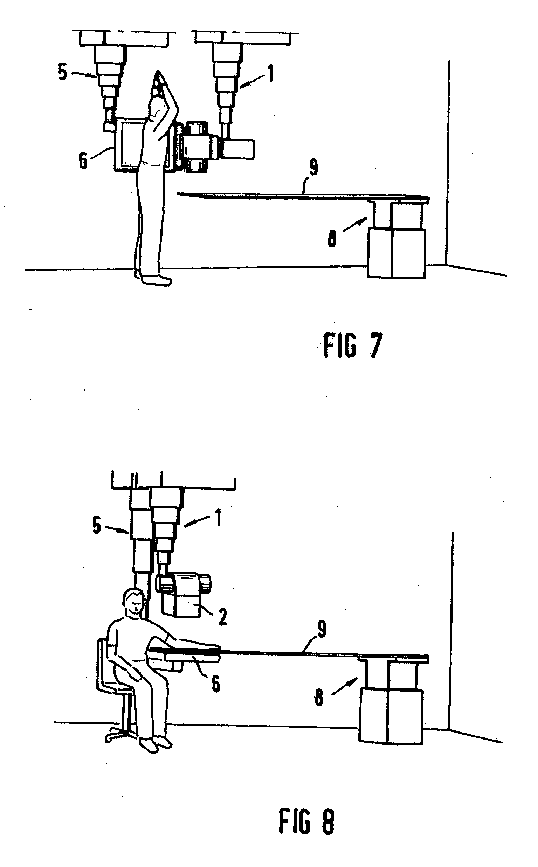

Die Fig. 7 zeigt die Anordnung von Röntgenstrahler und Strahlungsempfänger bei einer lateralen Thoraxaufnahme und die Fig. 8 schließlich die Positionierung der Deckenaufhängesysteme mit den daran befestigten Röntgenstrahlern bzw. Strahlungsempfängern bei einer Ellenbogenuntersuchung.

## Patentansprüche

1. Radiographie-System
mit einem ersten deckenaufgehängten System (1) für einen Röntgenstrahler (2), der in den drei Raumkoordinaten (x, y, z) mittels des ersten deckenaufgehängten Systems (1) verfahrbar und um wenigstens zwei Achsen (3, 4) schwenkbar ist,
mit einem zweiten, von dem ersten unabhängigen deckenaufgehängten System (5) für einen Strahlungsempfänger (6), der in den drei Raumkoordinaten (x, y, z) mittels des zweiten deckenaufgehängten Systems (5) verfahrbar ist, und
mit einem an dem zweiten deckenaufgehängten System (5) angebrachten Tragarm (10), an dem der Strahlungsempfänger (6) um wenigstens zwei Achsen (DHA, DVA, α, β) bewegbar aufgehängt ist,
wobei der Röntgenstrahler (2) und der Strahlungsempfänger (6) gegenüber positionierbar sind.

2. Radiographie-System nach Anspruch 1, **dadurch gekennzeichnet, dass** der Strahlungsempfänger (6) um wenigstens zwei horizontale Achsen (DHA, α, β) des Tragarms (10) bewegbar und um die senkrechte Achse (DVA) des zweiten deckenaufgehängten Systems (5) rotierbar aufgehängt ist.

3. Radiographie-System nach Anspruch1 oder 2, **dadurch gekennzeichnet, dass** der Strahlungsempfänger (6) ein digitaler Bildempfänger ist.

4. Radiographie-System nach einem der Ansprüche 1 bis 3, **dadurch gekennzeichnet, dass** der Röntgenstrahler (2) und der Strahlungsempfänger (6) mit motorischen Antrieben versehen sind und dass die mit Positionserfassungseinrichtungen versehenen Antriebe des Röntgenstrahlers (2) und des Strahlungsempfängers (6) über eine Einstellungs-Software miteinander gekoppelt sind.

5. Radiographie-System nach einem der Ansprüche 1 bis 4, **dadurch gekennzeichnet, dass** die Patientenlagerungsvorrichtung (8) eine einseitig aufgehängte bzw. abgestützte strahlungstransparente Lagerungsplatte (9) umfasst.

6. Radiographie-System nach einem der Ansprüche 1 bis 5, **dadurch gekennzeichnet, dass** die Deckenaufhängungen (1, 5) des Röntgenstrahlers (2) und des Bildempfängers (6) so ausgebildet sind, dass sie - in Gegenüberstellung - um den Patienten rotierbar sind.

7. Radiographie-System nach einem der Ansprüche 1 bis 6, **dadurch gekennzeichnet, dass** zur Aufnahme größerer Bereiche diese durch Verschieben des Strahlungsempfängers (6) bei gleichzeitiger Verschwenkung des Röntgenstrahlers (2) abgedeckt werden.

8. Radiographie-System nach Anspruch 7, **dadurch gekennzeichnet, dass** die Bildebene des Strahlungsempfängers (6) jeweils entsprechend der Verschwenkung des Zentralstahls (7) des Röntgenstrahlers (2) mitverkippt wird.
